# EUROPEAN PATENT SPECIFICATION

(11) **EP 1 048 275 B1**
(45) Date of publication and mention of the grant of the patent: **07.06.2006**
(21) Application number: 00108896.2
(22) Date of filing: 27.04.2000
(51) Int. Cl.: A61F 9/007

(54) **Apparatus for intubation of lacrimal duct**
Vorrichtung zur Intubation des Tränenkanals
Appareil d'intubation du canal lacrimal

(30) Priority: 28.04.1999 JP 12292799; 17.06.1999 JP 17045699; 26.08.1999 JP 23970699
(43) Date of publication of application: 02.11.2000
(73) Proprietor: MLC Limited Company, Hamamatsu-shi, Shizuoka-ken (JP)
(72) Inventor: Kurihashi, Katsuaki, Hamamatsu-shi, Shizuoka-ken (JP)
(74) Representative: Steil, Christian

(56) References cited:
- FR-A- 2 735 697
- US-A- 4 305 395
- DATABASE WPI Section PQ, Week 199816 Derwent Publications Ltd., London, GB; Class P32, AN 1998-172450 XP002170455 & JP 10 033584 A (MLC YG), 10 February 1998 (1998-02-10)

## Description

The present invention relates to an apparatus for intubation of a lacrimal duct comprising: a thin tube or rod; at least one thicker tube respectively extending from one end of the thin tube or rod, each of the thicker tubes defining an inner space and having a small cut; a probe insertable through the small cut of one of the at least one thicker tubes to enable the insertion of the thicker tubes and the thin tube or rod into the lacrimal duct.

Such an apparatus is known from JP 10033584 entitled "Pipe Insertion Implement for Lacrimal Tract Used in Treatment of Conjunctivities, Keratitis, Glaucoma - has thin Coupling with less Diameter to Couple End Members on both Sides of Central Member".

A further apparatus for intubation of a lacrimal duct is disclosed in document FR 2 735 697.

This invention generally relates to an apparatus for intubation of lacrimal duct (lacrimal drainage pathway) for treatment of lacrimal duct obstruction and so on.

A typical prior method of silicone intubation is shown in Japanese Patent Publication No. 56-50579.

In the prior method, the silicone tube is drawn into the lacrimal duct by a probe which is pulled out from the inferior nasal meatus.

Therefore, in the previous methods, a probe as a guide must be pulled out from the nostril. This maneuver is difficult. It is not rare that it takes a lot of time to retrieve the probes from the nostril or the retrieval is impossible.

For many years the present inventor has studied keenly for the treatment of lacrimal duct obstruction and dry eye, apparatus for intubation of the lacrimal duct which can be used easily with decreased pain to patients, can be quickly and correctly inserted into the lacrimal duct, is not easily dislocated during the intubation period, and can be easily removed after accomplishment of treatment.

As a result, various kinds of apparatus for intubation have been invented, for example, as shown in Japanese Patent Publication No.2811283, Japanese Patent Publication No. 2572340, Japanese Patent Publication No. 2539325, Japanese Patent Publication No. 8-19271, Japanese Patent Publication No. 8-30104, Japanese Patent Publication No. 9-276318, Japanese Patent Publication No. 2572352, Japanese Patent Publication No. 10-256109.

Above all, the apparatus shown in Japanese Patent Publication No. 2539325 is a typical invention made by the present inventor.

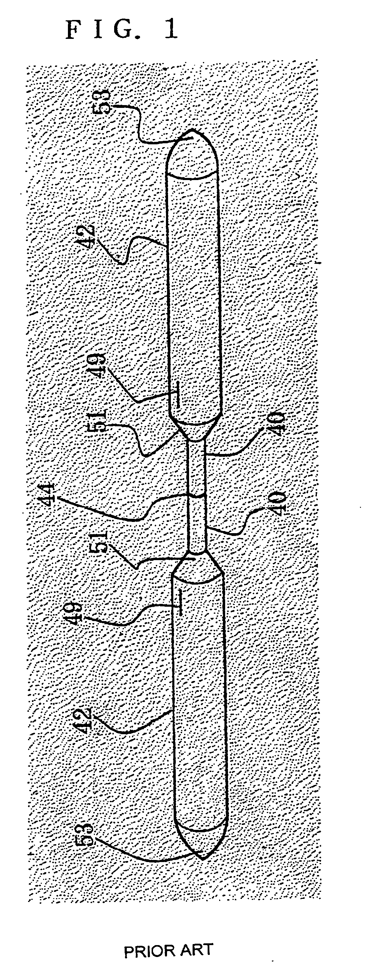

As shown in Figs. 1-3, in the typical prior art apparatus, the central part 20-40 mm of the tube 50-120 mm in length is thinner and softer, and the bilateral parts of the tube are thicker, and both ends are sharp pointed and closed. Thus, the apparatus for intubation of the lacrimal duct resembling a "nunchaku" type device which is used by Chinese martial art practitioners, has been developed.

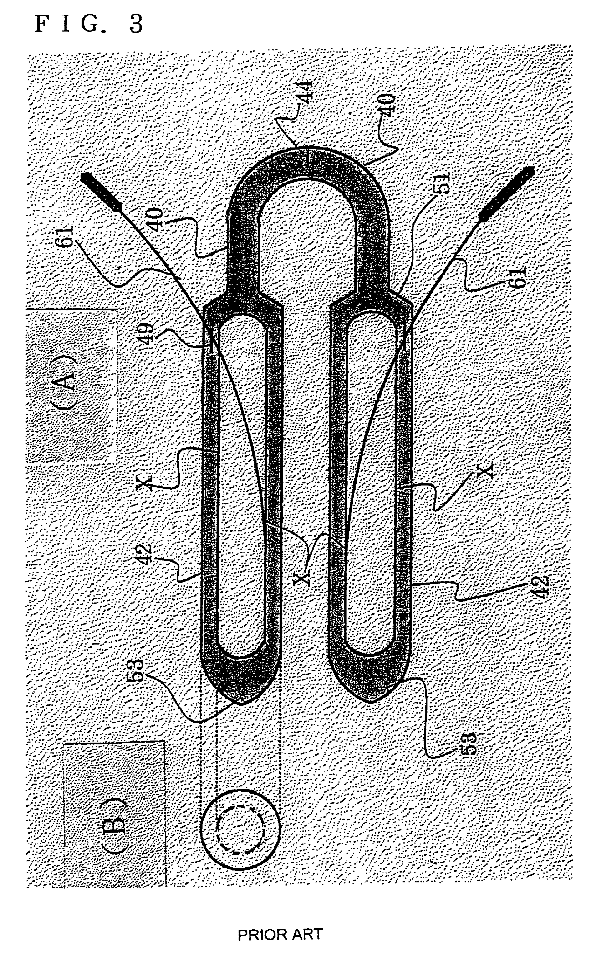

Fig. 3 (A) shows a cross sectional view showing a conventional nunchaku-style tubing.

As shown in Fig. 3, the thicker tube **42** is pushed into the lacrimal duct with the probe **61** which is inserted into the thicker tube. At the time, if there is too much lubricity between the probe **61** and the thicker tube **42**, the tube sometimes cannot be inserted into the lacrimal duct because the tip of the probe **61** slips on the inner surface **X** of the thicker tube, whereas, if the lubricity between the probe **61** and the thicker tube **42** is poor, the thicker tube sometimes turns back in the lacrimal duct together with the probe **61** when the probe **61** is removed from the thicker tube **42** (see for example, Kurihashi K: Dacryology, Medical A01 Shuppan, Inc, 1998, p.171).

One object of the present invention is to provide an apparatus which can be easily pushed into the lacrimal duct with the tip of a probe by making the inner surface of the thicker tube uneven.

Another object of the present invention is to provide an apparatus in which the probe can be easily removed from the thicker tube by making better lubricity between the probe and the thicker tube.

These objects are achieved by an apparatus for intubation of the lacrimal duct as described at the outset, wherein stopping means are formed in an inner surface of at least one of said thicker tubes for stopping a front end of said probe at least at a position between said small cut and a distal end of said one thicker tube.

By doing so, the thicker tube can be easily pushed into the lacrimal duct because the tip of the probe touches the step(s), protuberance(s), groove(s), other irregular surface or septum (septa). Furthermore lubricants such as olive oil, lipiodol ultrafluid and/or ointment are applied to the inner part of the thicker tube and/or probe. By doing so, better lubricity is provided between the thicker tube and the surface of the probe.

Although the columnar shape is good for the shape of the inner surface of the thicker tube, the frustum of the circular cone is better for the shape of the inner surface to operate the apparatus for intubation of the lacrimal duct. The shape of the frustum of the circular cone makes the thicker tube able to be pushed more effectively into the lacrimal duct.

The materials and shapes for the thicker tube and probe described in Japanese Patent Publication No. 2811283, Japanese Patent Publication No. 2572340, Japanese Patent Publication No.2539325, Japanese Patent Publication No. 2572352, Japanese Patent Publication No. 8-19271, Japanese Patent Publication No. 8-30104, Japanese Patent Publication No. 9-276318, Japanese Patent Publication No. 10-256109, can be used for the present invention.
Fig. 1 is a perspective view showing a conventional nunchaku-style tube.
Fig. 2 is a perspective view showing a conventional nunchaku-style tube.
Fig. 3A is a sectional view showing a conventional nunchaku-style tube shown in Fig. 2.
Fig. 3B is an end elevational view showing one end of a conventional nunchaku-style tube shown in Fig. 2.
Fig. 4A is a sectional view showing lubricant(s) applied to an apparatus according to the present invention.
Fig. 4B is an explanatory diagram showing lubricant(s) applied to a probe according to the present invention.
Fig. 5 is a sectional view showing an apparatus for intubation according to the present invention.
Fig. 6 is a sectional view showing an apparatus for intubation according to the present invention.
Fig. 7 is a sectional view showing an apparatus for intubation according to the present invention.
Fig. 8 is a sectional view showing an apparatus for intubation according to the present invention.
Fig. 9A is a sectional view showing an apparatus for intubation according to the present invention.
Fig. 9B is an end elevational view showing one end of an apparatus for intubation according to the present invention.
Fig. 10A is a sectional view showing an apparatus for intubation according to the present invention.
Fig. 10B is an end elevational view showing one end of an apparatus for intubation according to the present invention.
Fig. 11A is a sectional view showing an apparatus for intubation according to the present invention.
Fig. 11B is an end elevational view showing one end of an apparatus for intubation according to the present invention.
Fig. 12A is a sectional view showing an apparatus for intubation.
Fig. 12B is an end elevational view showing one end of an apparatus for intubation.
Fig. 13A is a sectional view showing an apparatus for intubation according to the present invention.
Fig. 13B is an end elevational view showing one end of an apparatus for intubation according to the present invention.
Fig. 14A is a sectional view showing other apparatus for intubation according to the present invention.
Fig. 14B is an end elevational view showing one end of an apparatus for intubation according to the present invention.
Fig. 15A is a sectional view showing other apparatus for intubation.
Fig. 15B is an end elevational view showing one end of an apparatus for intubation.
Fig. 16 is a sectional view showing other apparatus for intubation according to the present invention.
Fig. 17A is a sectional view showing other apparatus for intubation according to the present invention.
Fig. 17B is an end elevational view showing one end of an apparatus for intubation according to the present invention.
Fig. 18 is a sectional view showing other apparatus for intubation.
Fig. 19 is a sectional view showing other apparatus for intubation.
Fig. 20 is an explanatory diagram showing bicanalicular intubation according to the present invention.
Fig. 21 is a perspective view showing an apparatus for monocanalicular intubation shown in Japanese Patent Publication No.10-256109.
Fig. 22 is an explanatory diagram showing monocanalicular intubation shown in Japanese Patent Publication No.10-256109.
Fig. 23A is a perspective view showing an apparatus for intubation according to the present invention.
Fig. 23B is an end elevational view showing one end of an apparatus for intubation according to the present invention.
Fig. 23C is an end elevational view showing one end of an apparatus for intubation according to the present invention.
Fig. 24A is a perspective view showing an apparatus for intubation according to the present invention.
Fig. 24B is an end elevational view showing one end of an apparatus for intubation according to the present invention.
Fig. 24C is an end elevational view showing one end of an apparatus for intubation according to the present invention.
Fig. 25 is a perspective view showing an apparatus for intubation according to the present invention.
Fig. 26A is a sectional view showing an apparatus for intubation according to the present invention.
Fig. 26B is an and elevational view showing one end of an apparatus for intubation according to the present invention.
Fig. 26C is an and elevational view showing one end of an apparatus for intubation according to the present invention.
Fig. 27A is a sectional view showing an apparatus for intubation according to the present invention.
Fig.27B is an end elevational view showing one end of an apparatus for intubation according to the present invention.
Fig. 27C is an end elevational view showing one end of an apparatus for intubation according to the present invention.
Fig. 28A is a sectional view showing an apparatus for intubation according to a present invention.
Fig. 28B is an end elevational view showing one end of an apparatus for intubation according to the present invention.
Fig. 28C is an end elevational view showing one end of an apparatus for intubation according to the present invention.
Fig. 29A is a sectional view showing an apparatus for intubation according to the present invention.
Fig. 29B is an end elevational view showing one end of an apparatus for intubation according to the present invention.
Fig. 29C is an end elevational view showing one end of an apparatus for intubation according to the present invention.
Fig. 30A is a sectional view showing an apparatus for intubation.
Fig. 30B is an and elevational view showing one end of an apparatus for intubation.
Fig. 31 is an explanatory diagram showing a previous apparatus for intubation.

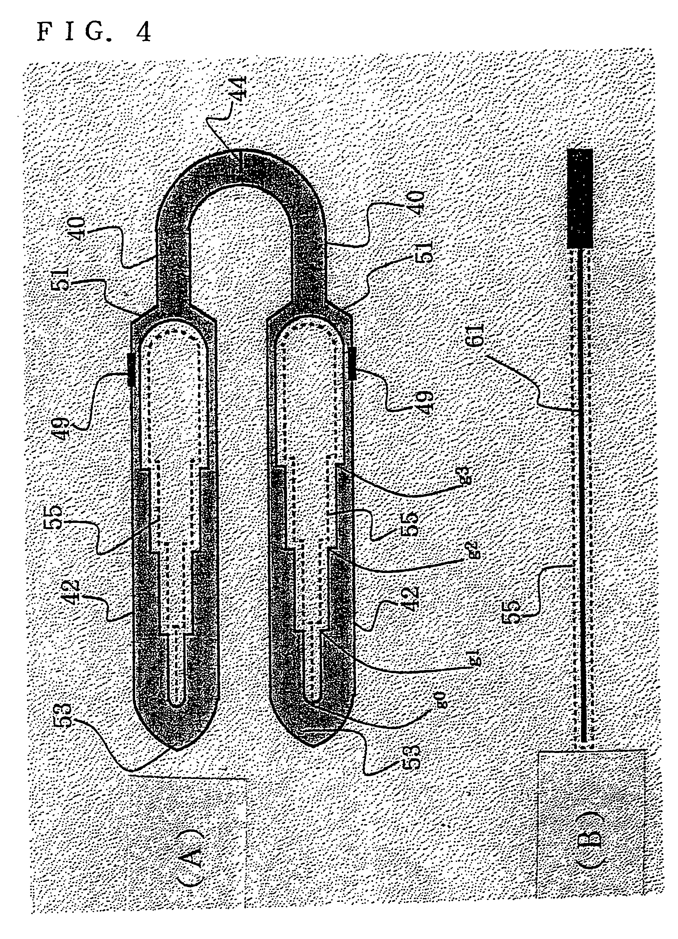

As shown in Fig. 4(A), thicker tubes are connected with both ends of the thinner tube (or rod), while the inner surface of the thicker tube has steps **g1**, **g2**, **g3** and the tip of the thicker tube **42** is sharp pointed, conical in shape and closed.

As shown in Fig. 4(A) (B), lubricant **55** such as olive oil, lipiodol ultrafluid, and/or ointment is applied to the inner part of the thicker tube and/or probe in advance.

Small cuts (1.5-1.0mm in length) **49** for the probe (0.25-0.4mm in diameter) **61** to insert, are made. The small cuts **49** are applied to the thicker tubes parallel to the axis of the tube **42**. The positions of the small cuts **49** are near the other end of the thicker tube **42**. Markings corresponding to the positions of the small cuts make it easier to discover the small cuts.

The length and thickness of the tube depend on the length of the lacrimal duct and size of the inner space of the individual lacrimal ducts. The present invention (85-105mm in total length) which consists of the central thinner tube (or rod) **40** (0.5mm in thickness and 25mm in length), and bilateral thicker tube **42**, (1mm in outer diameter, 0.5mm in inner diameter and 30~40mm in length) is most frequently used.

Although in order for the tube to be stable in the lacrimal duct, it is important that the central segment of the tube is thinner and softer, other optional shapes of it are also useful.

The middle point **44** of the central thinner segment is marked.

The thicker tubes **42** are connected with both ends of the thinner tube (or rod) with silicone adhesive.

As shown in Fig. 4, it is better to make a slope at the junction **51** between the thicker tube **42** and the thinner tube (a rod) **40**.

As shown in Fig. 4, if the tip **53** of the thicker tube **42** is sharp pointed and in conical shapes, it is more easily inserted from the lacrimal puncta.

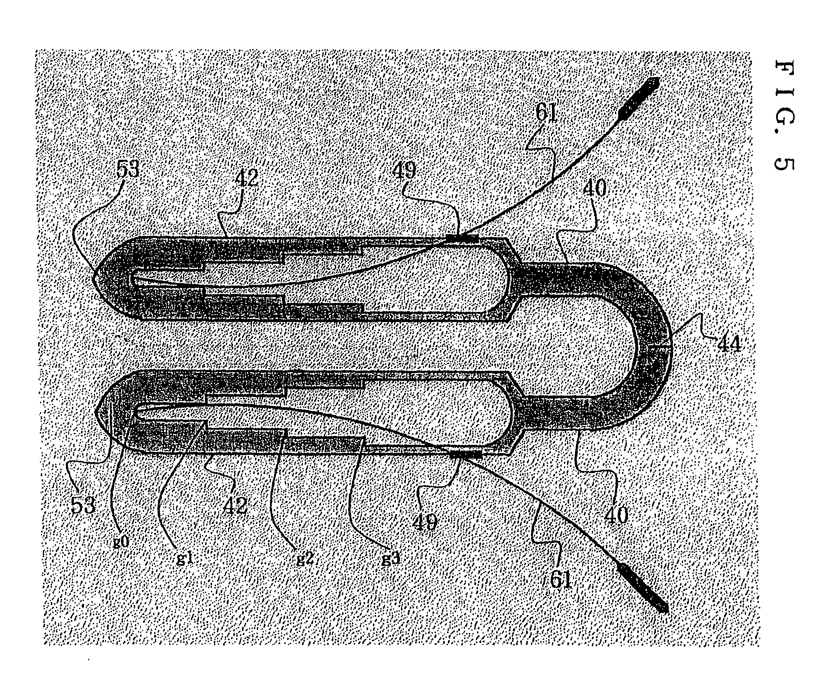

As shown in Fig. 5, the thicker tubes **42** equipped with probes **61** in advance are more convenient.

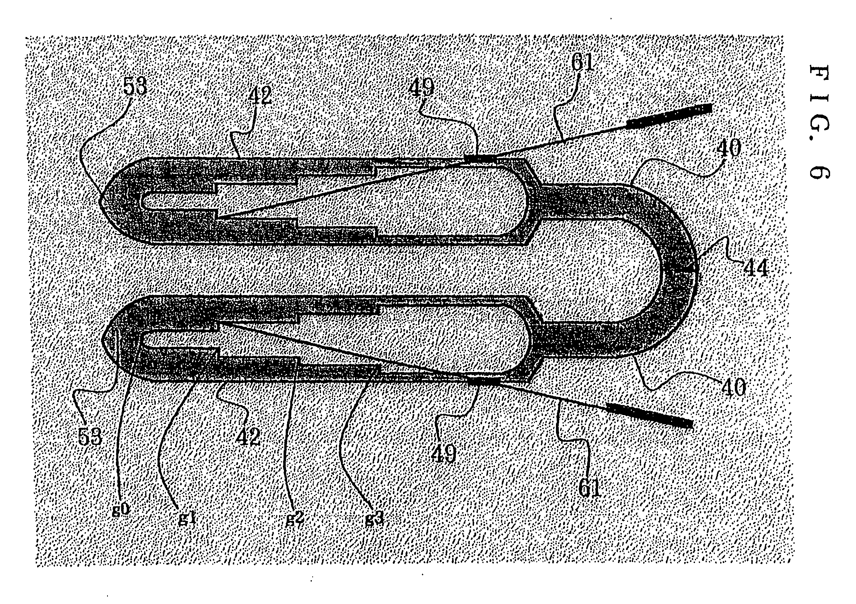

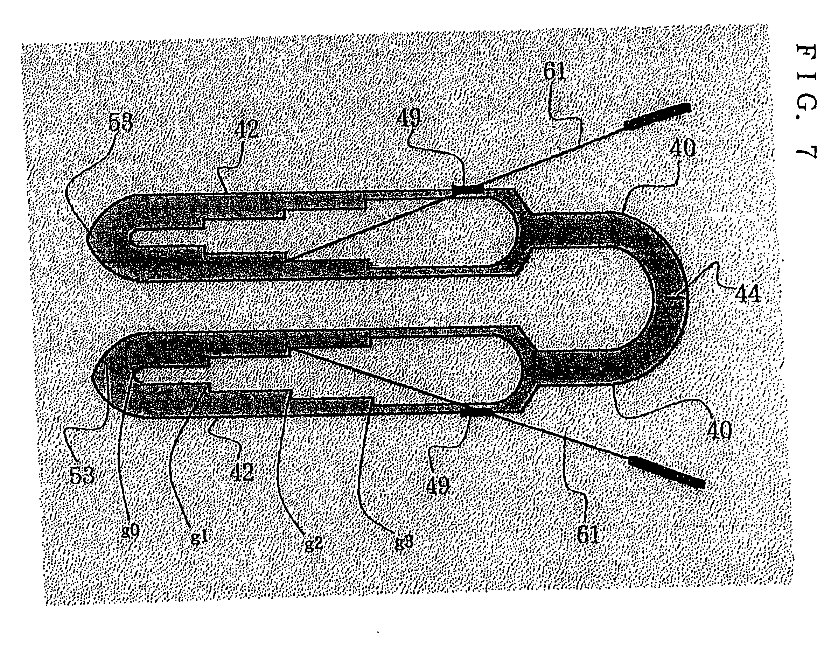

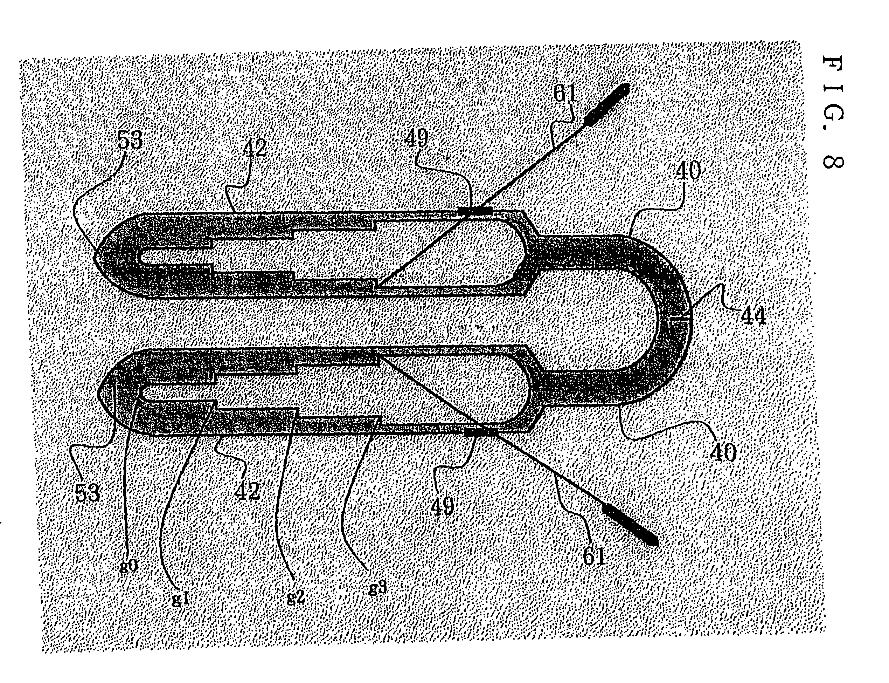

As shown in Figs. 6, 7 and 8, the thicker tubes **42** can be pushed into the lacrimal duct by pushing the steps **g1**, **g2** and **g3** on the inner surface of the thicker tubes **42** with the tip of the probe **61.**

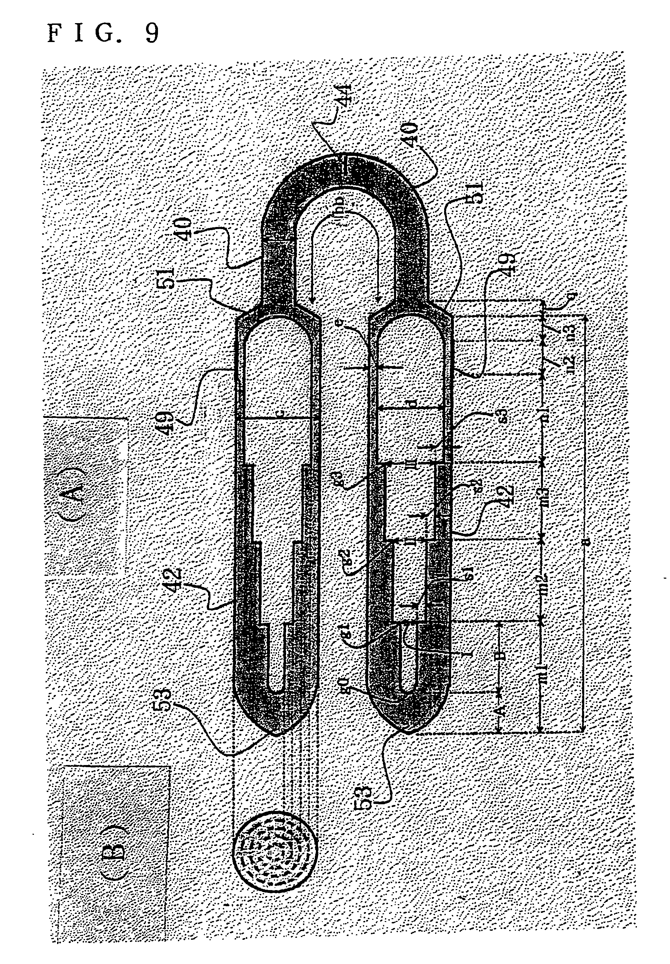

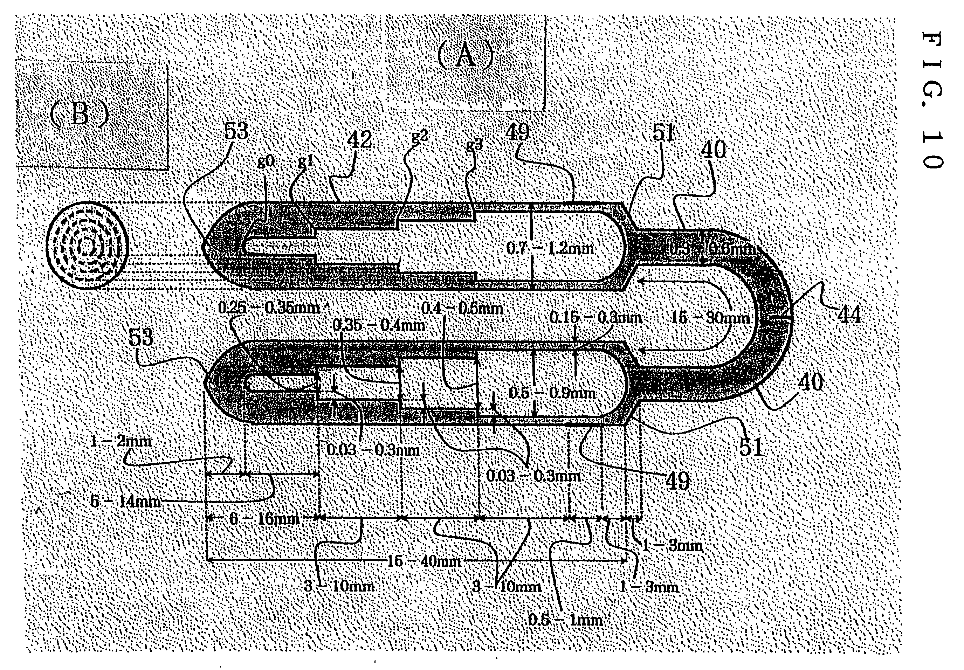

As shown in Figs. 9 and 10, the length **a** of the thicker tube is 15-40mm, the length **bb** of the thinner tube (or rod) is 15-30mm, and the length **q** of the boundary portion **51** between the thicker tube **42** and the thinner tube (or rod) is 1-3mm.

As shown in Figs. 9 and 10, the outer diameter **c** of the thicker tube is 0.7-1.2mm, the inner diameter **I** is 0.25-0.35mm, the inner diameter **II** is 0.35-0.4mm, the inner diameter **III** is 0.4-0.5mm, and the inner diameter **d** is 0.5-0.9mm. The outer diameter **f** of the thinner tube (or rod) is 0.3-0.6mm.

As shown in Figs. 9 and 10, the length **A** from the tip to closed end **g0** is 1-2mm, the length **B** from the closed end **g0** to step **g1** is 5-14mm, and the length **m1** from the tip to step **g1** is 6-16mm, the length **m2** from the step **g1** to step **g2** is 3-10mm, the length **m3** from the step **g2** to step **g3** is 3-10mm, the length **n1** from the step **g3** to small cut **49** is 3-10mm, the length **n2** of the small cut is 0.5-1mm, and the length **n3** from the small cut to other end of the thicker tube is 1-3mm.

As shown in Figs. 9 and 10, the wall thickness **e** of the thicker tube **42** is 0.15-0.3mm, and the height **s1** of the step is 0.03-0.3mm in any of the steps **S1, S2** and **S3.**

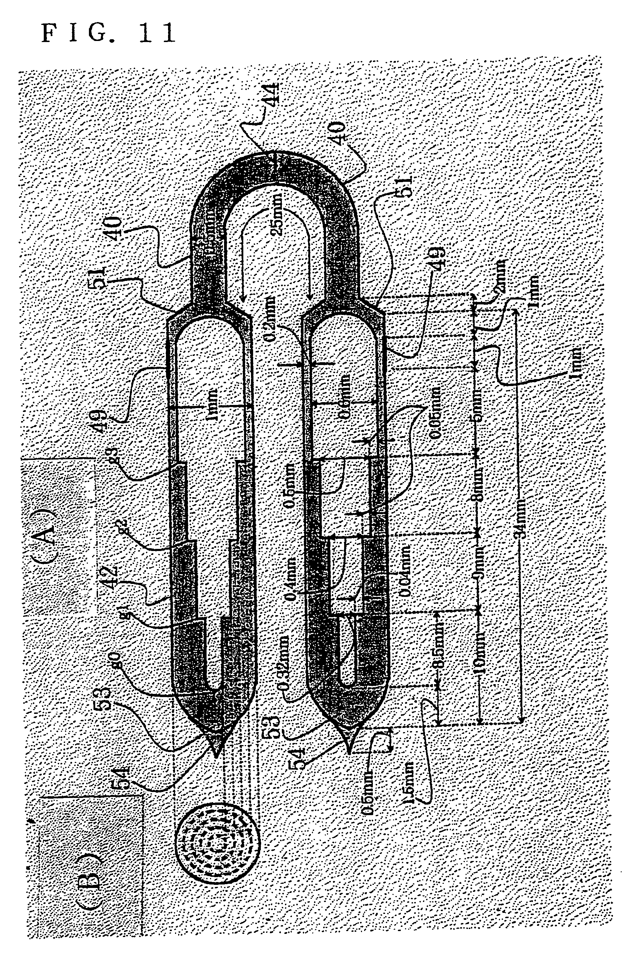

Fig. 11 shows a typical embodiment among many with step(s) shown in Figs. 9 and 10. Although in Figs. 9, 10 and 11, the number of steps in the thicker tube **42** is three: **g1**, **g2** and **g3**, more or less than three is also useful. The tip **53** is pointed as shown at **54**.

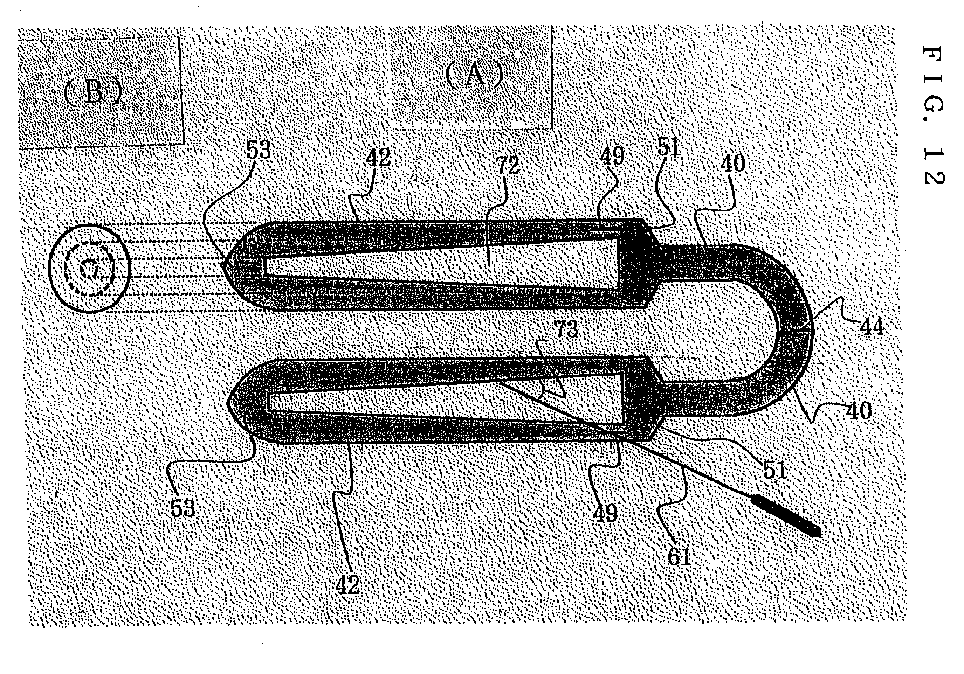

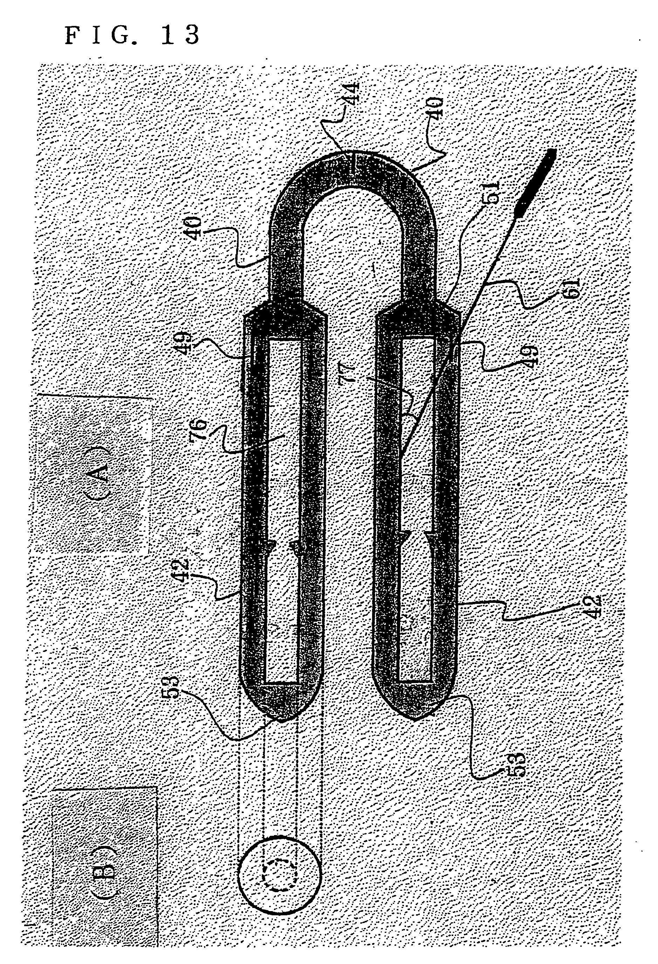

As shown in Fig. 12, if the shape of the inner space of the thicker tube is the frustum **72** of the circular cone, a probe **61** can be pushed into the lacrimal duct easier. The reason for this is that because the angle **73** formed by the inner surface of the tube and the probe shown in Fig. 12 is greater than the angle **77** shown in Fig. 13, the tip of the probe can press the inner surface more effectively. The inner space **76** is cylindrical.

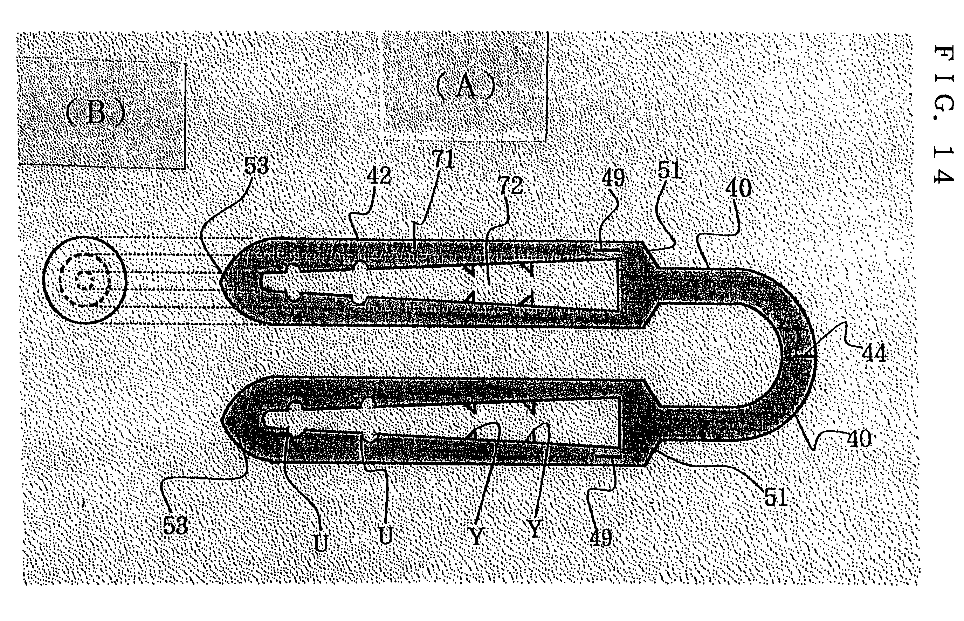

As shown in Fig. 14, if the protuberances **Y** and grooves **U** are applied to the inner surface **72** with the shape of the frustum of the circular cone of the thicker tube, the thicker tube **42** can be pushed into the lacrimal duct by the probe **61** more easily than the apparatus for intubation shown in Figs. 12. Other irregular inner surface (not illustrated) is also useful for the thicker tube **42**. The body of the thicker tube **42** is shown at 71.

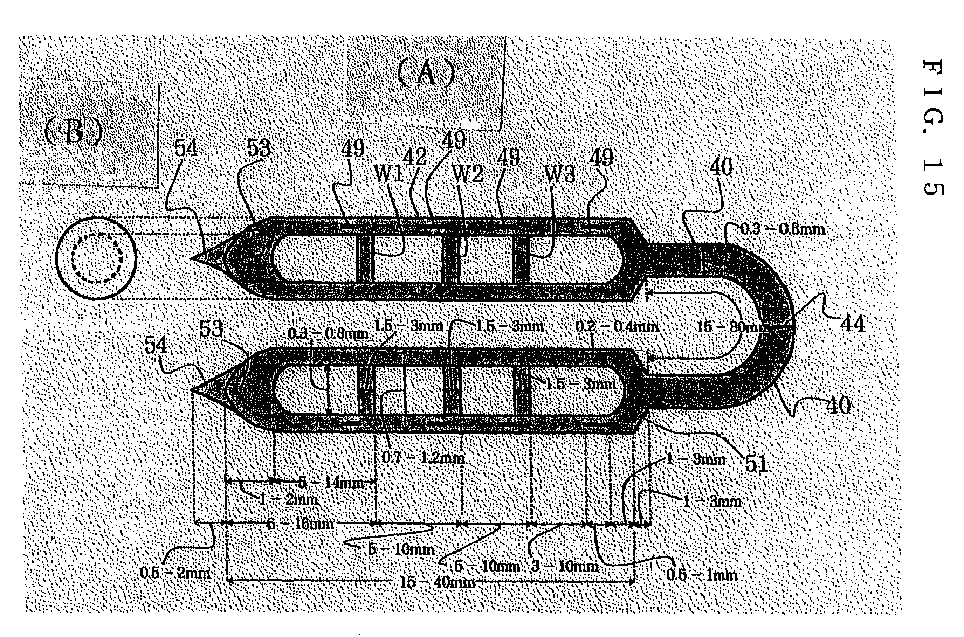

As shown in Fig. 15, by setting the septa **W1**, **W2** and **W3** in the thicker tubes like knot(s) of bamboo, the thicker tubes can be inserted into the lacrimal duct securely and easily by using a probe **61**.

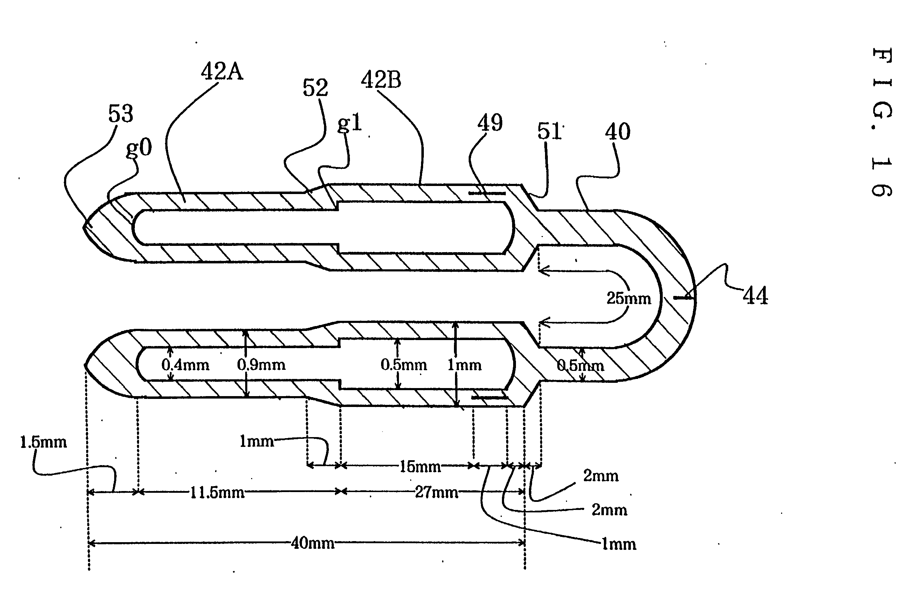

In the embodiment of Fig. 16 with a typical size, the thicker tube **42** consists of the anterior portion **42A** and posterior portion **42B**. The outer diameter of the anterior portion **42A** is 0.1-0.3 mm smaller than that of the posterior portion **42B**. There is a slope **52** and step **g1** between the two different sized tubes **42A** and **42B**.

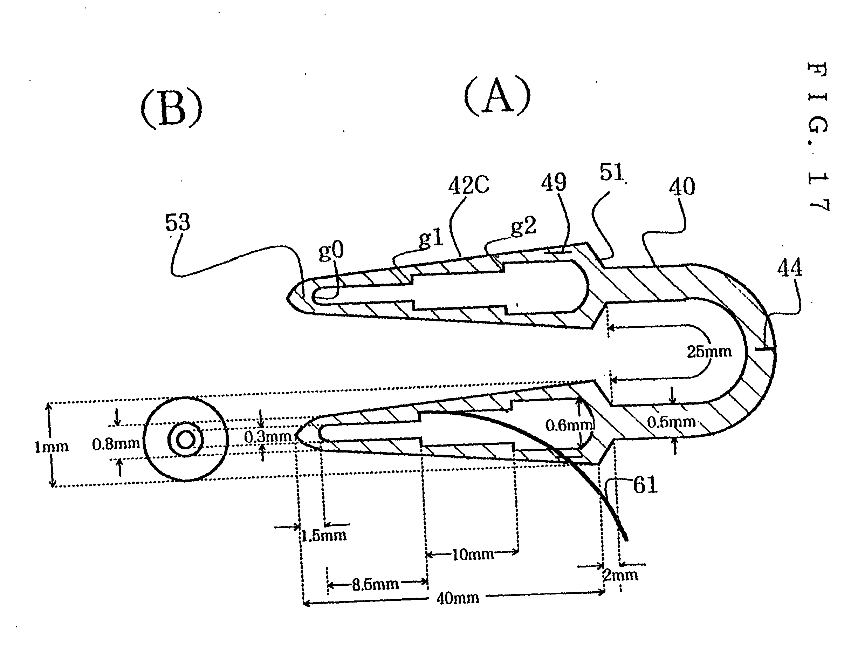

In the embodiment shown in Fig. 17 with a typical size, the carrot thicker tubes **42C** are in the shape of a carrot and have the steps **g1**, **g2**. The different sized tubes **42A**, **42B** in Fig. 16 and the carrot thicker tubes **42C** in Fig. 17 can be more easily inserted into the lacrimal duct than the thicker tube **42** in Figs. 1-15.

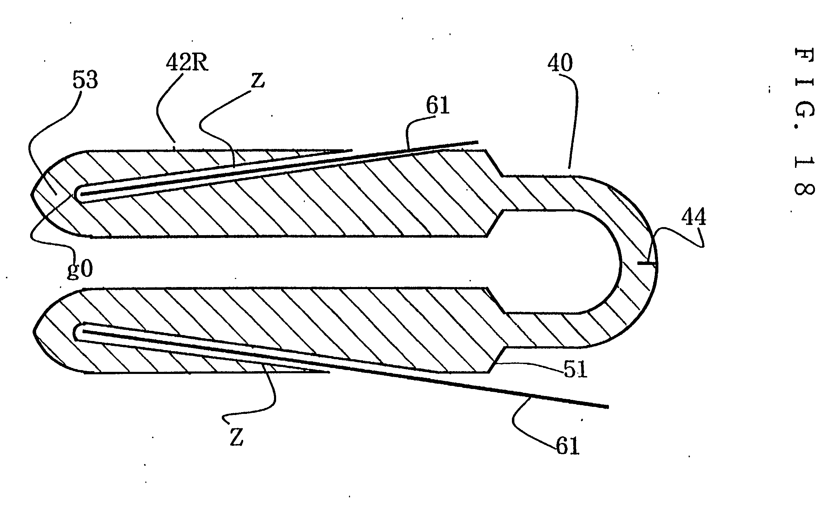

As shown in Fig. 18, the thicker rods **42R** are connected with a thinner rod **40**. The thicker rods **42R** have a hole **Z** for a probe **61** to be inserted. The thicker rods **42R** have the same size and shape as the thicker tube in Figs. 1-15. The hole has its entrance preferably at the outer side of rod **42R**.

The different sized thicker rods which have the same size and shape as the different sized thicker tubes **42A**, **42B** are also useful (not illustrated).

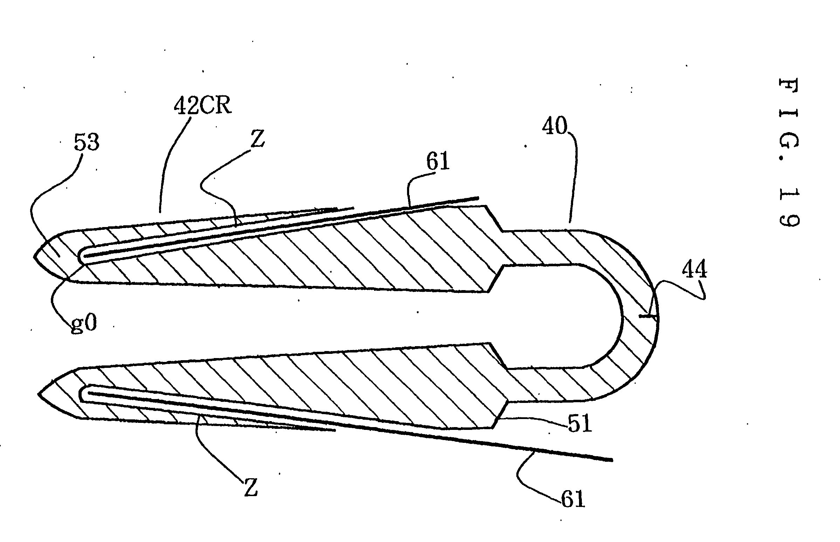

As shown in Fig. 19, the carrot thicker rods **42CR** are connected with the thinner rod **40**. The carrot thicker rods **42CR** are in the shape of a carrot and have a hole **Z** for a probe to be inserted.

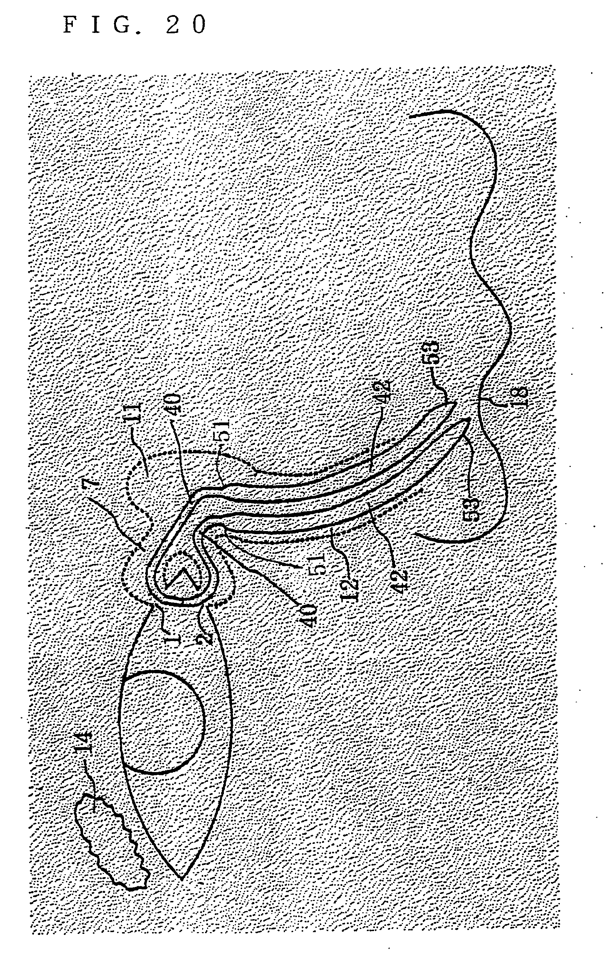

Fig. 20 shows an instance of the state of the present invention which is inserted into the lacrimal duct.

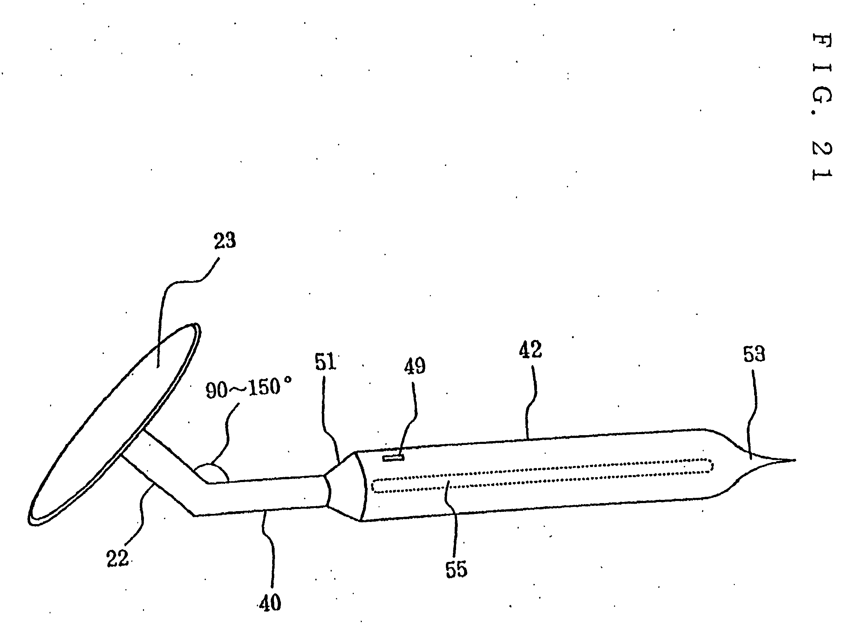

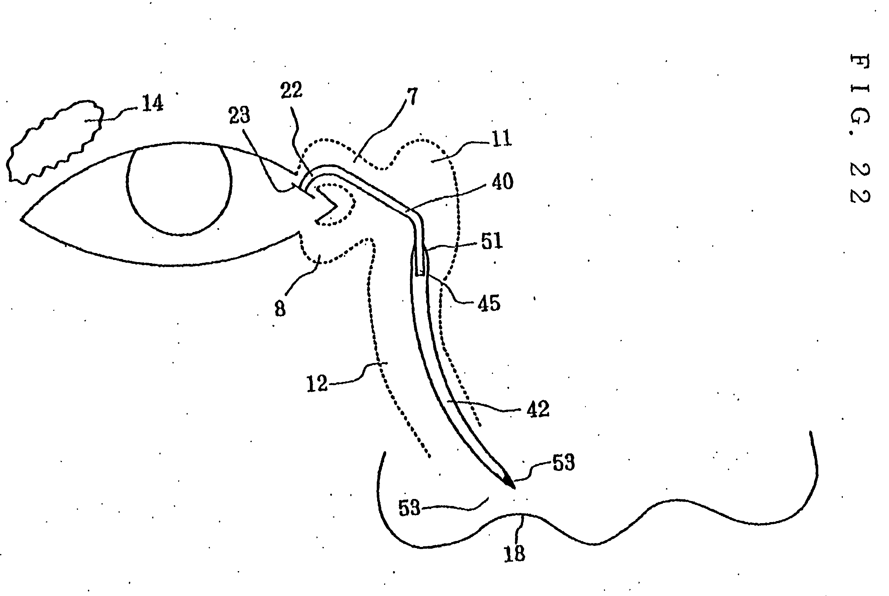

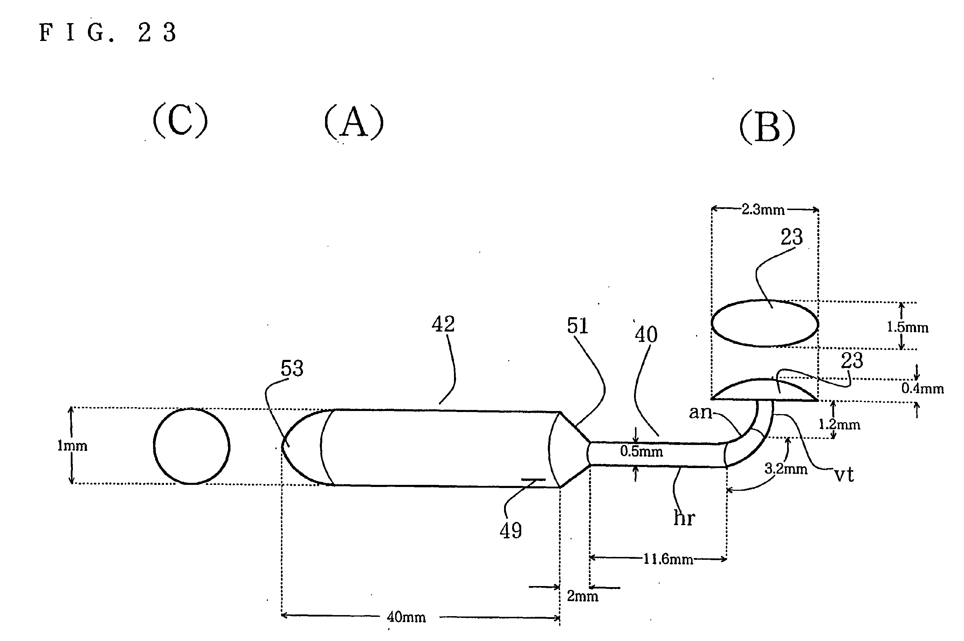

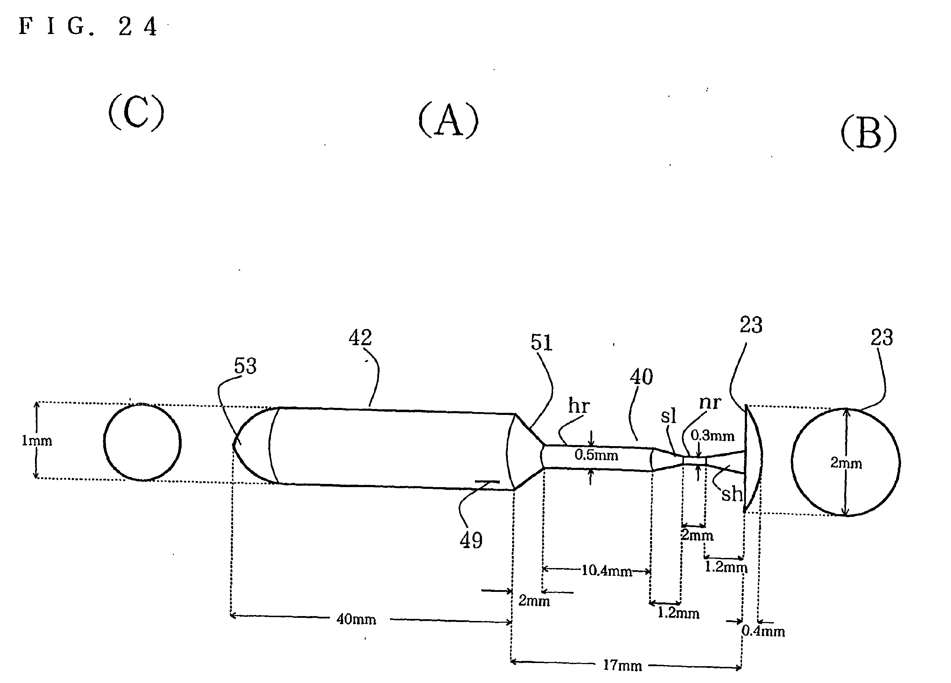

The thicker tubes (or rods) **42, 42C, 42A, B, 42R, 42CR** in Figs. 4-19 can be used as the thicker tube **42** for monocanalicular intubation, for example, in Figs. 21 and 22 as shown in Japanese Patent Publication No.10-256109 and Figs. 23 and 24 with typical size and shape which are other apparatus for monocanalicular intubation according to the present invention.

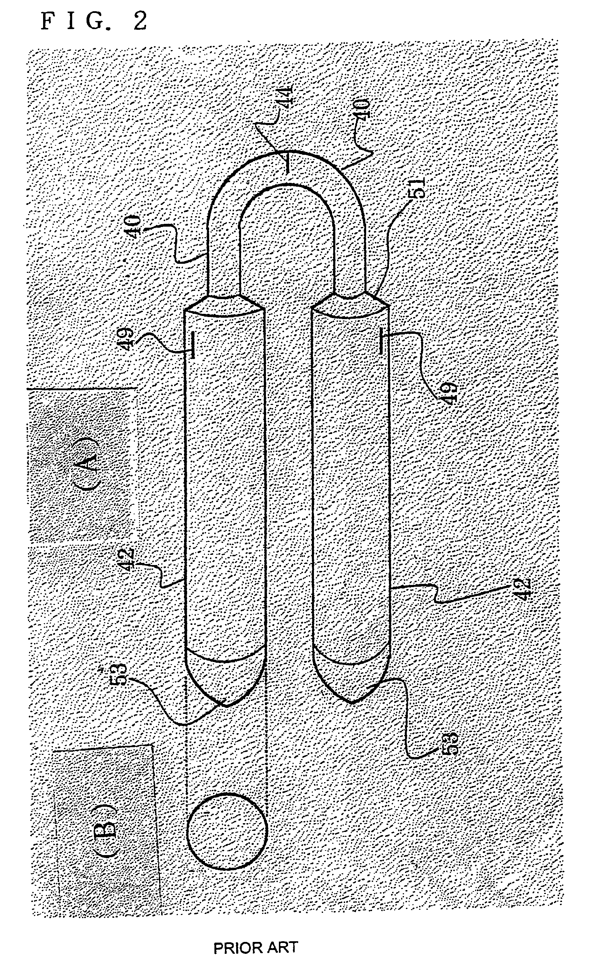

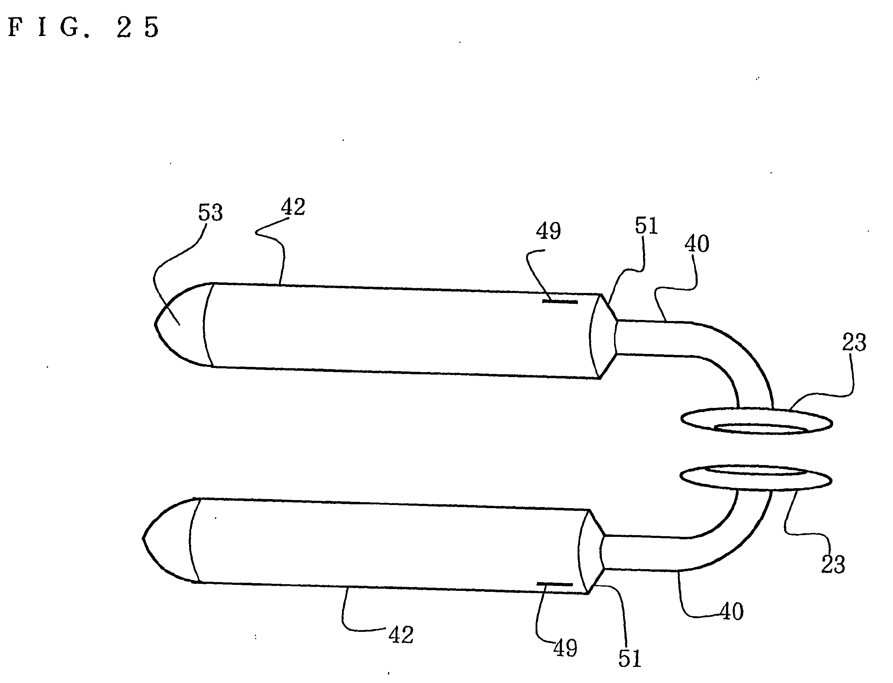

As shown in Fig. 25, the apparatus in Figs. 21-24 are designed based on the nunchaku style tubing in Fig. 2 and consists of a half part of the nunchaku style tubing and a brim **23** which is glued with the end of the thinner tube (or rod) **40**.

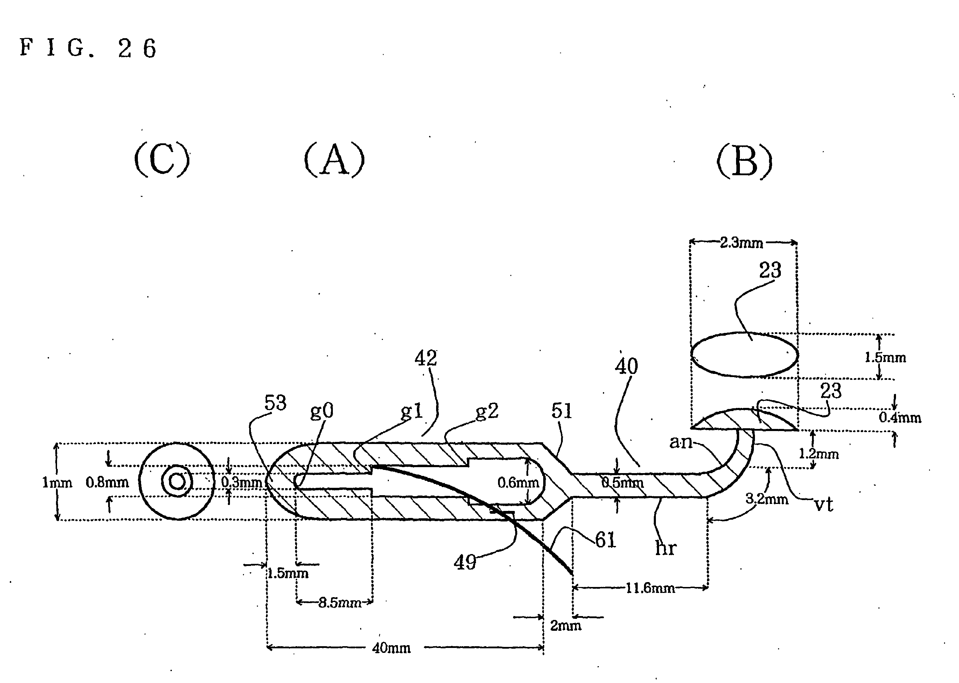

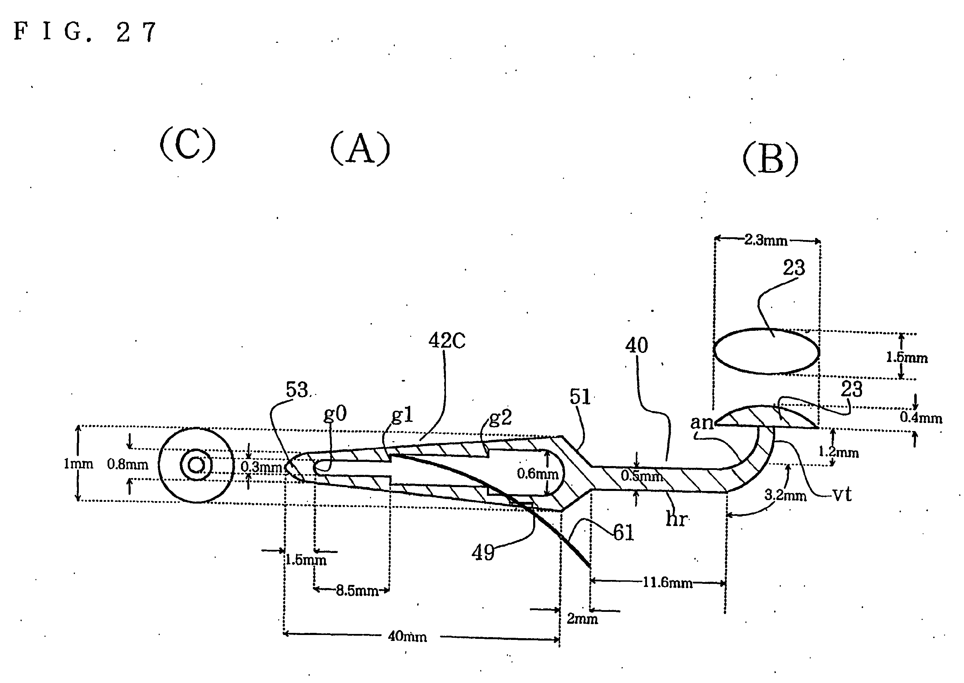

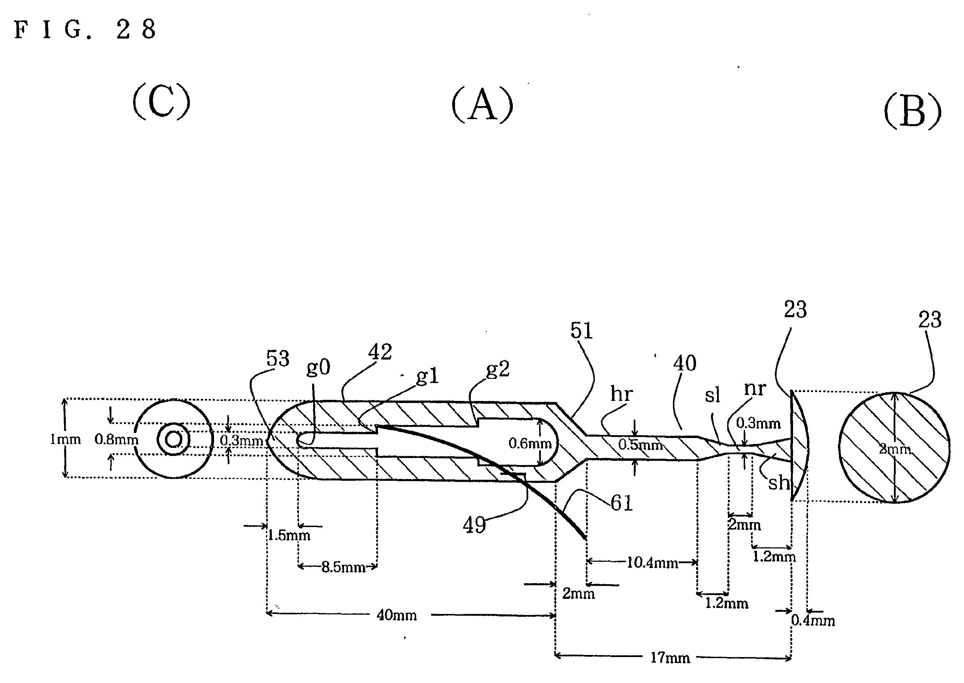

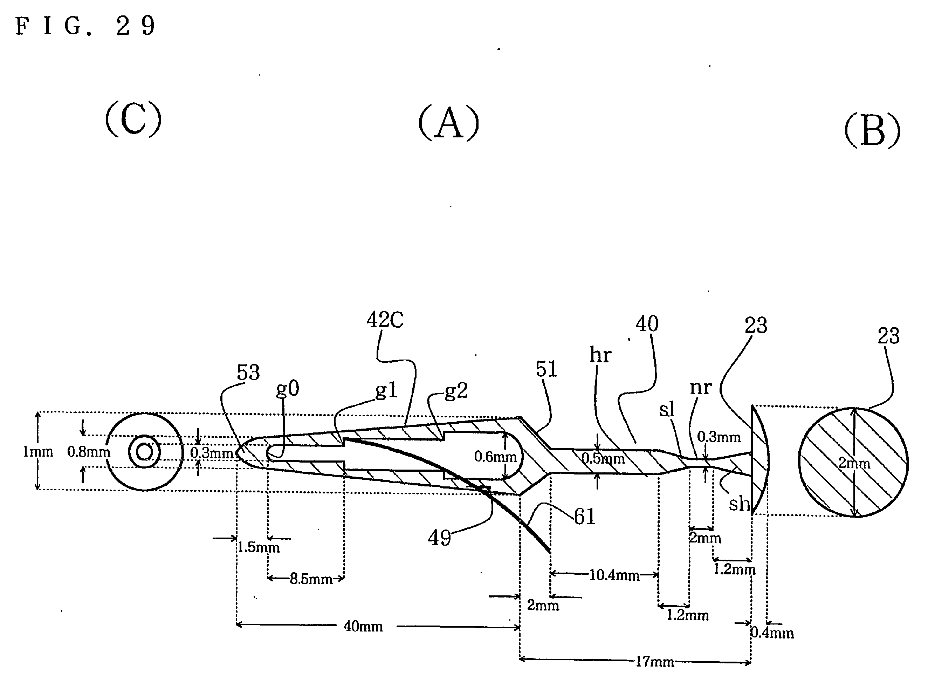

And Figs. 26-29 with typical size and shape show an apparatus for monocanalicular intubation according to the present invention. In the embodiments shown in Figs. 23, 26 and 27, the thinner rod **40** 16mm in length consists of the horizontal segment **hr** 11.6mm in length, curved segment **an** 3.2mm in length and vertical segment **vt** 1.2mm in length and the end of the latter is glued with the brim **23**.

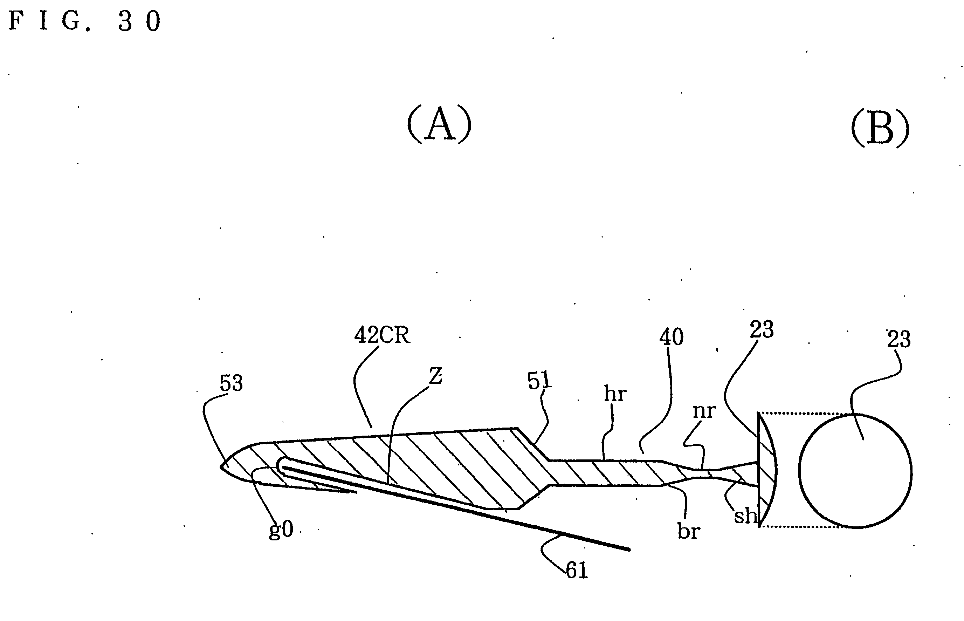

In Figs. 24 and 28-30 with typical size, the thinner rod **40** 15mm in length consists of the horizontal segment **hr** 10.4mm in length, slope **sl** 1.2mm in length, narrow segment **nr** 2mm in length and short segment **sh** 1.2mm in length.

Fig. 22 shows an instance of the state of the present invention in Figs. 23-29 which is inserted into the lacrimal duct from the upper punctum.

As shown in Figs. 20 and 22, the boundary portion **51** must be positioned in the lacrimal sac - nasolacrimal duct for the stability of the apparatus in the lacrimal duct.

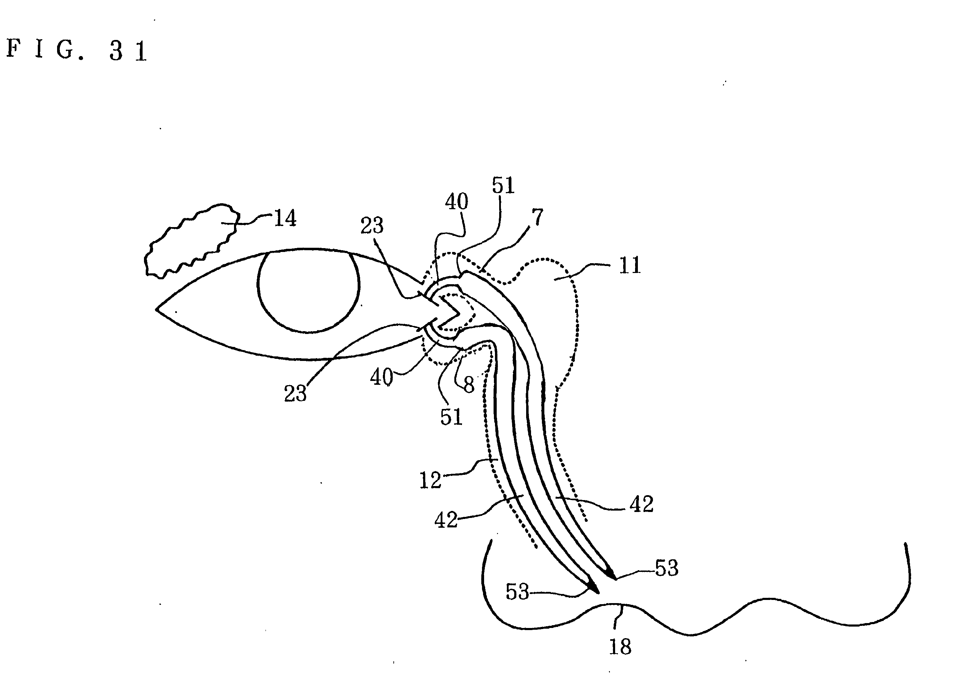

The canalicular length of an adult is 10-15mm with an avarage of 11mm (for example see Kurihashi K:Dacryology, Medical A01 shuppan Inc., Tokyo, 1998). Therefore, the thinner tube (or rod) **40** in Fig. 20 must be 20-30 mm or more in length for adults, and the thinner tube (or rod) **40** in Fig. 22 must be 10-15mm or more for adults. If the boundary portion **51** is positioned in the canaliculus as shown in Fig. 31, the apparatus are not stable in the lacrimal duct and easily dislocate.

The length of the thinner tube (or rod) in Fig. 22 is more than the canalicular length to get better stability in the lacrimal duct. By doing so, the boundary portion **51** between the thinner tube (or rod) **40** and thicker tube (or rod) **42** positioned in the lacrimal sac - nasolacrimal duct **11, 12** which runs perpendicularly and slight posteriorly and thinner tube (or rod) **40** in the horizontal part of the canaliculus **7,8** which runs horizontally and is pulled by the thicker tube (or rod) with the help of gravity, results in good stability in the lacrimal duct without fixation like the nunchaku style tube shown in Fig.20.

The thicker tube (or rod) **42** is heavier than the thinner tube (or rod) **40**. With the pulling effect of the thicker tube (or rod) **42**, the brim **23** in Figs. 21-30 presses the punctum and prevents tear fluid from entering the punctum (the opening of the canaliculus).

The apparatus in Figs. 4-30 can be used as a drug delivery system or in conjunction with a drug delivery system as in the conventional nunchaku-style tube in Figs. 1-3, and the brim **23** in Figs. 23-30 can be used as the ocusert which is a drug delivery system for glaucoma, or other drug delivery system (For example see Kurihashi K: Japanese Patent Publication No.9-276318).

Apparatus for intubation of the lacrimal duct can be easily inserted into the lacrimal duct, resulting in a short operating time and small burden on patients.

## Claims

1. An apparatus for intubation of a lacrimal duct (11, 12) comprising:
a thin tube or rod (40);
first and second thicker tubes (42) extending from both ends of the thin tube or rod (40), each of the thicker tubes (42) having an inner space (72) and a small cut (49);
a probe (61) insertable through the small cut (49) of one of the thicker tubes (42) to enable the insertion of the thicker tubes (42) and the thin tube or rod (40) into the lacrimal duct (11, 12),
**characterized by**
stopping means formed in an inner surface of at least one of said thicker tubes (42) for stopping a front end of said probe (61) at least at a position between said small cut (49) and a distal end of said one thicker tube.

2. The apparatus of claim 1, **characterized in that** at least the one thicker tube (42) has a shape of a frustrum of a circular cone.

3. The apparatus of claim 1 or 2, **characterized in that** the stopping means is a step (g1, g2, g3), and the longer the distance from the tip (53), the larger the inner space of the thicker tubes (42) becomes.

4. The apparatus of any of claims 1-3, **characterized in that** the stopping means are a soft and elastic protuberance(s)(Y), and/or groove(s)(U).

5. The apparatus of any of claims 1-4, **characterized in that** the thicker tubes (42) consist of two different sized tubes (42A, 42B) in outer diameter.

6. The apparatus of any of claims 1-4, **characterized in that** the thicker tubes (42) are in the shape of a carrot (42C).

7. The apparatus of any of claims 1-6, **characterized in that** lubricant (55) is applied to the inner part of the thicker tube (42) and/or to probe (61).

8. The apparatus of any of claims 1-7, **characterized in that** boundary portions (51) between the thin tube (40) and thicker tubes (42) are positioned in the lacrimal sac - nasolacrimal duct.

9. An apparatus for intubation of a lacrimal duct (11, 12) comprising:
a thin tube or rod (40);
only one thicker tube (42) extending from one end of the thin tube (40), the thicker tube (42) having an inner space and a small cut (49);
a probe (61) insertable through the small cut (49) of the thicker tube (62) to enable the insertion of the thicker tube (42) and the thin tube or rod (40) into the lacrimal duct (11, 12);
**characterized by**
stopping means formed in an inner surface of said thicker tube (42) for stopping a front end of said probe (61) at least at a position between said small cut (49) and a distal end of said one thicker tube.

10. The apparatus of claim 9, **characterized in that** the thin tube or rod (40) consists of horizontal (hr), curved (an) and vertical (vt) segments.

11. The apparatus of claim 9, **characterized in that** the thinner tube or rod (40) consists of horizontal (hr), slope (sl), narrow (nr) and short (sh) segments.

12. The apparatus of any of claims 9-11, **characterized in that** a brim (23) is connectable to a drug delivery system.

## Patentansprüche

1. Vorrichtung zur Intubation eines Tränenkanals (11, 12), mit:
einem dünnen Rohr bzw. Stab (40);
einem ersten dickeren Rohr und einem zweiten dickeren Rohr (42), die sich von beiden Enden des dünneren Rohrs bzw. Stabs (40) erstrecken, wobei jedes der dickeren Rohre (42) einen Innenraum (72) und einen kleinen Schlitz (49) aufweist;
eine Sonde (61), die über den kleinen Schlitz (49) eines Rohrs der dickeren Rohre (42) einführbar ist, um die Einführung der dickeren Rohre (42) und des dünneren Rohrs bzw. Stabs (40) in den Tränenkanal (11, 12) zu ermöglichen,
**gekennzeichnet durch**
Anschlagmittel, die in einer Innenfläche von zumindest einem der dickeren Rohre (42) ausgebildet sind, um ein vorderes Ende der Sonde (61) zumindest an einer Stelle zwischen dem schmalen Schlitz (49) und einem distalen Ende des einen dickeren Rohrs aufzuhalten.

2. Vorrichtung nach Anspruch 1, **dadurch gekennzeichnet, dass** zumindest das eine dickere Rohr (42) die Form eines kreisförmigen Kegelstumpfs aufweist.

3. Vorrichtung nach Anspruch 1 oder 2, **dadurch gekennzeichnet, dass** die Anschlagmittel eine Stufe (g1, g2, g3) darstellen und je länger die Entfernung von der Spitze (53) ist, desto größer wird der Innenraum des dickeren Rohrs (42).

4. Vorrichtung nach einem der Ansprüche 1 bis 3, **dadurch gekennzeichnet, dass** die Anschlagsmittel eine weiche und elastische Protuberanz bzw. Protuberanzen (Y) und/oder Vertiefung bzw. Vertiefungen (U) ist bzw. sind.

5. Vorrichtung nach einem der Ansprüche 1 bis 4, **dadurch gekennzeichnet, dass** das dickere Rohr (42) aus zwei Rohren (42A, 42B) mit einem unterschiedlichen äußeren Durchmesser gebildet wird.

6. Vorrichtung nach einem der Ansprüche 1 bis 4, **dadurch gekennzeichnet, dass** die dickeren Rohre (42) die Form einer Karotte (42C) aufweisen.

7. Vorrichtung nach einem der Ansprüche 1 bis 6, **dadurch gekennzeichnet, dass** in dem inneren Teil des dickeren Rohrs (42) und/oder der Sonde (61) ein Schmiermittel verwendet wird.

8. Vorrichtung nach einem der Ansprüche 1 bis 7, **dadurch gekennzeichnet, dass** sich Grenzteile (51) zwischen dem dünnen Rohr (40) und den dicken Rohren (42) in dem Tränensack - Tränennasengang befinden.

9. Vorrichtung zur Intubation eines Tränenkanals (11, 12), mit:
einem dünnen Rohr bzw. Stab (40);
lediglich einem dickeren Rohr (42), das sich von einem Ende des dünneren Rohrs (40) erstreckt, wobei das dickere Rohr (42) einen Innenraum und einen kleinen Schlitz (49) aufweist;
einer Sonde (61), die durch den kleinen Schlitz (49) des inneren Rohrs (62) einführbar ist, um die Einführung des dickeren Rohrs (42) und des dünneren Rohrs bzw. Stabs (40) in den Tränenkanal (11, 12) zu ermöglichen;
**gekennzeichnet durch**
Anschlagmittel, die in einer Innenfläche des dickeren Rohrs (42) zum Aufhalten eines vorderen Endes der Sonde (61) bei zumindest einer Stelle zwischen dem kleinen Schlitz (49) und einem distalen Ende des einen dickeren Rohrs gebildet sind.

10. Vorrichtung nach Anspruch 9, **dadurch gekennzeichnet, dass** das dünne Rohr bzw. der Stab (40) aus horizontalen (hr), gebogenen (an) und vertikalen (vt) Segmenten gebildet ist.

11. Vorrichtung nach Anspruch 9, **dadurch gekennzeichnet, dass** das dünnere Rohr bzw. der Stab (40) aus horizontalen (hr), abgestuften (s1), engen (nr) und kurzen (sh) Segmenten gebildet ist.

12. Vorrichtung nach einem der Ansprüche 9 bis 11, **dadurch gekennzeichnet, dass** ein Rand (23) mit einem Drogenzuführsystem verbindbar ist.

## Revendications

1. Appareil pour l'intubation d'un canal lacrymal (11, 12) comprenant :
un tube fin ou tige (40);
des premier et second tubes plus épais (42) s'étendant à partir des deux extrémités du tube fin ou tige (40), chacun des tubes plus épais (42) ayant un espace interne (72) et une petite découpe (49) ;
une sonde (61) pouvant être insérée par la petite découpe (49) de l'un des tubes plus épais (42) pour permettre l'insertion des tubes plus épais (42) et du tube fin ou tige (40) dans le canal lacrymal (11, 12),
**caractérisé en ce qu'**il comprend :
des moyens de butée formés dans une surface interne d'au moins l'un desdits tubes plus épais (42) pour arrêter une extrémité avant de ladite sonde (61) au moins à une position située entre ladite petite découpe (49) et une extrémité distale dudit un tube plus épais.

2. Appareil selon la revendication 1, **caractérisé en ce qu'**au moins le un tube plus épais (42) a une forme d'un tronc conique d'un cône circulaire.

3. Appareil selon la revendication 1 ou 2, **caractérisé en ce que** les moyens de butée sont un palier (g1, g2, g3), et plus la distance à partir de la pointe (53) est longue, plus l'espace interne des tubes plus épais (42) devient important.

4. Appareil selon l'une quelconque des revendications 1 à 3, **caractérisé en ce que** les moyens de butée sont une (des) protubérance(s) souple(s) et élastique(s) (Y) et/ou une (des) rainure(s) (U).

5. Appareil selon l'une quelconque des revendications 1 à 4, **caractérisé en ce que** les tubes plus épais (42) se composent de deux tubes (42A, 42B) dimensionnés différemment du point de vue du diamètre externe.

6. Appareil selon l'une quelconque des revendications 1 à 4, **caractérisé en ce que** les tubes plus épais (42) ont une forme de carotte (42C).

7. Appareil selon l'une quelconque des revendications 1 à 6, **caractérisé en ce que** du lubrifiant (55) est appliqué sur la partie interne du tube plus épais (42) et/ou de la sonde (61).

8. Appareil selon l'une quelconque des revendications 1 à 7, **caractérisé en ce que** les parties de limite (51) situées entre le tube fin (40) et les tubes plus épais (42) sont positionnées dans le sac lacrymal - canal nasolacrymal.

9. Appareil pour l'intubation d'un canal lacrymal (11, 12) comprenant :
un tube fin ou tige (40) ;
un seul tube plus épais (42) s'étendant à partir d'une extrémité du tube fin (40), le tube plus épais (42) ayant un espace interne et une petite découpe (49) ;
une sonde (61) pouvant être insérée dans la petite découpe (49) du tube plus épais (62) pour permettre l'insertion du tube plus épais (42) et du tube fin ou tige (40) dans le canal lacrymal (11, 12) ;
**caractérisé en ce qu'**il comprend :
des moyens de butée formés dans une surface interne dudit tube plus épais (42) pour arrêter une extrémité avant de ladite sonde (61) et moins à une position située entre ladite petite découpe (49) et une extrémité distale dudit un tube plus épais.

10. Appareil selon la revendication 9, **caractérisé en ce que** le tube fin ou tige (40) se compose de segments horizontaux (hr), incurvés (an) et verticaux (vt).

11. Appareil selon la revendication 9, **caractérisé en ce que** le tube plus fin ou tige (40) se compose de segments horizontaux (hr), inclinés (sl), étroits (nr) et courts (sh).

12. Appareil selon l'une quelconque des revendications 9 à 11, **caractérisé en ce qu'**un bord (23) peut être raccordé à un système d'administration de médicament.
